(19) **Europäisches Patentamt**

**European Patent Office**

**Office européen des brevets**

(11) **EP 1 221 614 B1**

(12) **EUROPEAN PATENT SPECIFICATION**

(45) Date of publication and mention
of the grant of the patent:
**18.01.2006 Bulletin 2006/03**

(51) Int Cl.:
*G01N 33/49* *(2006.01)*     *B01L 3/00* *(2006.01)*

(21) Application number: **01309783.7**

(22) Date of filing: **21.11.2001**

(54) **Instrument and method for blood separation, and preparing method, quantifying method and preserving container for biological sample**

Vorrichtung und Verfahren zur Bluttrennung und Vorbereitungsverfahren, Quantifizierungsverfahren und Konservierungsbehälter für eine biologische Probe

Appareil et méthode pour la séparation du sang et méthode de préparation, méthode de quantification et récipient conservateur pour un échantillon biologique

(84) Designated Contracting States:
**AT BE CH CY DE DK ES FI FR GB GR IE IT LI LU MC NL PT SE TR**
Designated Extension States:
**AL LT LV MK RO SI**

(30) Priority: **05.01.2001 JP 2001000830**

(43) Date of publication of application:
**10.07.2002 Bulletin 2002/28**

(73) Proprietor: **Leisure, Inc.**
**Shibuya-Ku,**
**Tokyo (JP)**

(72) Inventors:
• **Nanba, Hiromi**
**Nakano-Ku,**
**Tokyo (JP)**
• **Horita, Masatoshi**
**Shimabara-Shi,**
**Nagasaki-Ken (JP)**
• **Koga, Osamu**
**Minamitakaki-Gun,**
**Nagasaki-Ken (JP)**
• **Ohta, Yoshio**
**Okayama-Shi,**
**Okayama-Ken (JP)**

(74) Representative: **Skone James, Robert Edmund**
**GILL JENNINGS & EVERY,**
**Broadgate House,**
**7 Eldon Street**
**London EC2M 7LH (GB)**

(56) References cited:
WO-A-91/08782     WO-A-96/20402
WO-A-97/29369     US-A- 5 304 348
US-A- 5 624 597

**Description**

The present invention relates to a preparing method of a sample for quantification used for clinical diagnosis and a method for quantifying elements to be quantified in a biological sample by using the sample for qualification prepared by said preparing method.

Generally, a blood collection is categorised into a normal blood collection where a specifically qualified person, such as a doctor, collects blood from a vein by using a syringe, and a self-blood collection where a test subject collects blood in person by pricking a finger of his or her hand with a blood collecting needle.

Conventionally the blood collected by normal blood collection system is carried to a test station as contained in an airtight container, where the blood is tested after it has been separated into blood cell and blood plasma by a centrifugal separator. On the other hand, the blood collected by self-blood collection system is carried to the test station as dried after impregnated into a filter paper, where an analyses is conducted in a way that a white part of the filter paper representative of the blood plasma is cut off at the test station and then is dissolved into a solvent, leaving a red part thereof representative of the blood cell.

In a clinical inspection, a specifically qualified person, such as a doctor, a nurse and a clinical inspection engineer or an expert engineer collects a biological sample by the blood collection or the like, prepares a sample for quantification from the collected biological sample and quantifies an element to be quantified in a biological sample.

Although in a special inspection item for a qualitative or a semi-quantitative judgment, there has been known a method where a test subject collects his/her own biological sample by himself/herself, in a general inspection item for quantification, it has been required that the test subject goes to a hospital or an inspection center where the specifically qualified person, such as a doctor, a nurse and a clinical inspection engineer or the expert engineer is, or otherwise the specifically qualified person, such as a doctor, a nurse and a clinical inspection engineer or the expert engineer goes to a residence of the test subject to collect the biological sample from the test subject. In addition, an operation for preparing the sample for quantification from the collected biological sample has been performed by the specifically qualified person, such as a doctor, a nurse and a clinical inspection engineer or the expert engineer.

Since upon preparing the sample for quantification, it is required to quantify a specified volume thereof accurately, and accordingly this is rather troublesome, it has not been practiced that the test subject quantifies a specified volume of biological sample to prepare the sample for quantification or that the specifically qualified person or the expert engineer prepares the sample for quantification from the collected biological sample right on the spot of collection.

As a result of an improved performance of an automatic analyzer, a sample volume to be subjected to the inspection has been decreased, and accordingly such a large amount of biological sample as it has been is not now required to be collected. As the automatic analyzer using a method in which the element to be quantified is quantified by using a sample prepared by diluting a specified volume of biological sample by a specified volume of diluent solution is known Bio Majesty JCA-BA1650 (provided by JEOL).

However, in the normal blood collection system, since the supernatant blood plasma has to be sucked by a dropping pipet and transferred into a special container prepared for blood plasma analyses after the collected blood has been centrifugally separated, the operation for separating the blood into the blood cell and the blood plasma has taken rather long time, leading to higher cost, and further the operation for transferring the blood plasma into the special container has a potential risk of mix-up or the similar accidents.

Still further, due to the fact that the blood cell in the blood is hemolyzing as time goes by and the accuracy guarantee period of the blood left at ambient temperature is not more than one day, in the case where the test has been conducted after that period, there occurs some problems that affect test results or measured values to be no more available as a polestar for medical care and diagonosing, including that the measured values of electrolytic materials, such as sodium, kalium, chrome and the likes, might be adversely effected in their accuracy and the numeric values of enzyme system, such as GOT, GPT and the likes could not be measured.

Yet further, to separate the blood by a centrifugal separator and to conduct a test on predetermined items, the blood amount of about 5 to 10 ml is required for each collection. Accordingly, since it is difficult for a test subject in person to collect the blood, but a specifically qualified person, such as a doctor, has to collect the blood, the person of test subject is required to go to the hospital or the likes, or otherwise the qualified person has to go to the place of the person of test subject, thus requiring a long time and much labor for blood collection.

On the other hand, in the self-blood collection, since a series of steps is required comprising impregnating the blood into a filter paper, drying the filter paper, and then dissolving the filter paper into a solvent, the self-blood collection is usable to the test exclusively on such item that the test value could not be affected by going through those steps, but is not employable for the test on the other items.

In a manner of conventional clinical diagnosis, the specifically qualified person, such as a doctor, a nurse and a clinical inspection engineer or the expert engineer as well as the test subject have to bear heavy burden respectively, and a process from the collection of the biological sample to the practical inspection has been rather complicated. Accordingly, it has been desired to develop an inventive method of diagnosis in which the burden of the specifically qualified person, such as a doctor, a nurse and a clinical inspection engineer or the expert engineer as well as the test subject should be minimized, and to build up a simple clinical diagnosis system with said inventive method of diagnosis incorporated therein.

Accordingly, the present invention, in the light of the problems described above, provides an inventive instrument and method for separating blood, which accomplishes a reduction of cost in a blood test, a longer shelf life of the blood, and an improved test accuracy with a small amount of blood to be collected and a simplified operation.

Further, the present invention has been made to provide a method for preparing a sample for quantification from a biological sample, which is to be used for quantifying elements to be quantified in said biological sample; a method for quantifying the elements to be quantified in the biological sample; a container for preserving an unknown volume of biological sample containing elements to be quantified, which has been collected without quantifying a volume thereof, until it will be quantified; and a container used for preparing a sample for quantification from an unknown volume of biological sample containing elements to be quantified, which has been collected without quantifying a volume thereof.

The present invention is made in the light of the situations described above and has features below to solve the problems mentioned above.

US 5304348 discloses a device and method for optically analysing blood, wherein a known volume of a whole blood sample is mixed with a known volume of an aqueous diluent and subsequently the dilution ratio is determined.

The present invention provides a method for quantification from an unknown volume of a biological sample, as defined in claim 1.

The sample for quantification used in the present invention is a solution composed of a specified volume of aqueous solution containing a specified amount of indicating material and an unknown volume of biological sample. In this sample for quantification, said aqueous solution containing a specified amount of indicating material used to dissolve the unknown volume of biological sample thereinto will be hereafter referred to as a reference solution.

The sample for quantification may be prepared either by mixing an unknown volume of biological sample collected without quantifying a volume thereof with a specified volume of aqueous solution containing a specified amount of indicating material, or by adding a specified volume of aqueous solution containing a specified amount of chromogen to a solution made by mixing an unknown volume of biological sample and a specified volume of aqueous solution. Accordingly, since there is no need to use a container for quantifying a volume of the biological sample, the sample for quantification can be easily prepared right on the spot of collection of the biological sample. Further, very small amount of biological sample may be sufficient to prepare the sample for quantification.

The reference solution may be prepared either by mixing a specified amount of indicating material with a specified volume of aqueous solution or by adding a specified volume of aqueous solution containing a specified amount of chromogen to a specified volume of aqueous solution.

The biological sample may be obtained by a normal method without using a specific one, that is, for example, the blood serum may be obtained from the whole blood by, after having left it for a while, applying a centrifugal processing thereto, and the blood plasma may be obtained by treating the whole blood with a separation processing such as a membrane separation.

In the present invention, a self-blood collection method where, for example, a test subject collects his/her blood in person by manipulating a blood collecting needle may be used preferably. In addition, since this operation is performed without quantifying a volume thereof and accordingly no special technique is required for preparing the sample for quantification, the test subject can prepare the sample for quantification by himself/herself. Further, the sample for quantification prepared by mixing the whole blood directly with a specified volume of aqueous solution is used as a sample for determining a concentration of the elements to be quantified in the blood plasma or the blood serum from a value obtained by quantifying the elements to be quantified in the sample for quantification after, if necessary, having separated blood cell element by the separation processing such as a centrifugal separation and a membrane separation and a dilution ratio determined based on a dilution ratio calculation method described below.

There is no special limitation on a manner for mixing the biological sample with the specified volume of aqueous solution, but the sample obtained in the above-described method may be added directly or may be added indirectly through a separation means installed in a container. The latter adding method may include, for example, a method where by using a blood separation instrument, the blood plasma separated from the whole blood is added.

There is no special limitation on a range of the dilution ratio of the indicating material in the sample for quantification, but it is preferably 2 to 10, more preferably 2 to 50, and most preferably 2 to 20.

It is to be noted that if the specified volume of aqueous solution does not contain a specified amount of indicating material, after the biological sample having been mixed therewith, a specified volume of aqueous solution containing a specified amount of indicating material may be added thereto.

A process for quantifying the elements to be quantified in an unknown volume of biological sample comprises the steps of quantifying a concentration of the indicating material in a reference solution and a concentration of the indicating material in the sample for quantification to determine the dilution ratio of the biological sample, and quantifying a concentration of the elements to be quantified in the sample for quantification.

The concentration (X) of the elements to be quantified in the biological sample may be determined by an equation 1 as a function of the concentration (Y) of the elements to be quantified in the sample for quantification prepared by the above-described method and the dilution ratio (a) of the biological sample in the sample for quantification.

$$X = a\ Y \qquad\qquad (\text{eq. 1})$$

In the present invention, the dilution ratio can be determined as described below.

$$C_2 = M_1\ /\ (V_1 + V_2) \qquad\qquad (\text{eq. 2})$$

where, $C_2$ is the concentration of the indicating material in the sample for quantification, $V_1$ is the volume of the aqueous solution used for preparing the sample for quantification, $M_1$ is the amount of the indication material used therefor, and $V_2$ is the volume of the biological sample used therefor (note: $V_2$ is not measured).

On the other hand, the concentration $C_1$ of the indicating material in the aqueous solution (= the reference solution) used for preparing the sample for quantification can be represented as:

$$C_1 = M_1\ /\ V_1 \qquad\qquad (\text{eq. 3})$$

It is to be noted that in the method for preparing the sample for quantification from the biological sample, the reference solution is a solution prepared without using the biological sample.

Since the dilution ratio (a) of the unknown volume of biological sample in the sample for quantification can be represented as:

$$a = (V_1 + V_2)\ /\ V_2 \qquad\qquad (\text{eq. 4})$$

the dilution ratio (a) may be rewritten by using $C_1$ and $C_2$ as shown in equation 5.

$$\text{Dilution ratio (a)} = (V_1 + V_2)\ /\ V_2 = C_1\ /\ (C_1 - C_2) \qquad (\text{eq. 5})$$

Herein, the concentrations $C_1$ and $C_2$ of the indicating material can be determined by measuring an absorptivity when the indicating material is a pigment or a chromogen, by measuring a light emitting intensity when the indicating material is a light emitting material, or by measuring a fluorescence intensity when the indicating material is a fluorescent material. When the indicating material is quantified by the absorptivity, since the concentration is in proportion to the absorptivity, they may be written as:

$$C_2\ /\ C_1 = E_2\ /\ E_1 \qquad\qquad (\text{eq. 6})$$

where, $C_1$ and $E_1$ are the concentration and the absorptivity of the reference solution, and $C_2$ and $E_2$ are those of the sample for quantification, respectively. Accordingly, the dilution ratio can be determined also by an equation rewritten as:

$$\text{Dilution ratio (a)} = C_1\ /\ (C_1 - C_2) = E_1\ /\ (E_1 - E_2) \qquad (\text{eq. 7})$$

As having been described above, the dilution ratio can be calculated based on the $C_1$ and $C_2$ values or the $E_1$ and $E_2$ values. It is to be noted that although the $C_1$ or the $E_1$ value may be set in advance to a known value, since they may be quantified by using the newly prepared solution, the amount of the indicating material in the reference solution may not be set in advance to a known value. That is, in the present invention, a volume of a solution to be mixed directly with the biological sample, and if this solution contains the indicating material, an amount or a concentration of the indicating material and a volume of the solution containing the indicating material and an amount or a concentration of the indicating material used for preparing the sample for quantification, each of them may not be known but arbitrary so far as it is constant.

The method for quantifying the indicating material may be any method so far as it can quantify the concentration of the indicating material. When the indicating material is a pigment, the absorptivity of the sample for quantification itself can be quantified. Further, in other cases, a specified volume of sample is taken out of the sample for quantification and then the concentration thereof is quantified by a quantifying method for the indicating material to be quantified. Upon quantification, when the absorptivity is used therefor, the value of absorptivity can be used directly without converting it to the concentration of the indicating material.

In the present invention, any one of a colorimetric method, a light emitting method and a fluorometric method may be used, and among them, the colorimetric method is most preferable.

The indicating material to be used in the colorimetric method may include, for example, the above-described pigments and chromogens. The chromogens may include the reductive coloring type chromogen and the oxidative coloring type chromogen. The colorimetric method using the reductive coloring type chromogen may include a method in which the reductive coloring type chromogen is converted into the pigment by an operation of an electron carrier of a reductive coenzyme such as NAD(P)H or the like, dihydrolipoamide dehydrogenase, 1-methoxy-5-methylphenaziummethylsulfate or the like and then the absorptivity of the generated pigment is measured by a spectrophotometer. The colori-metric method using the oxidative coloring type chromogen may include a method in which the oxidative coloring type chromogen is converted into the pigment by an operation of active material of peroxide such as hydrogen peroxide, peroxidase or the like and then the absorptivity of the generated pigment is measured by the spectrophotometer. In the case where the chromogen is used, the method using the oxidative coloring type chromogen is preferable.

When the chromogen is used as the indicating material, the chromogen is converted into the pigment by the method described below and then the absorptivity of the generated pigment is measured. In the case where the reductive coloring type chromogen is used, the reductive coloring type chromogen is converted into the pigment by the operation of the electron carrier of the reductive coenzyme such as NAD(P)H or the like, dihydrolipoamide dehydrogenase, 1-methoxy-5-methylphenaziummethylsulfate or the like and then the absorptivity of the generated pigment is measured. In the case where the oxidative coloring type chromogen is used, the oxidative coloring type chromogen is converted into the pigment by the operation of the active material of peroxide such as hydrogen peroxide, peroxidase or the like and then the absorptivity of the generated pigment is measured.

The fluorometric method may include a method in which the fluorescence emitted from the above-described fluorescent material by the active material of peroxide such as hydrogen peroxide, peroxidase or the like is measured by a fluorophotometer.

The fluorometric method may further include another method in which the light (photon) emitted from the above-described fluorescent material by the active material of peroxide such as hydrogen peroxide, peroxidase or the like is measured by a luminometer.

It is to be noted that in the case where the coupling type chromogen is used as the oxidative coloring type chromogen, either one of two compounds pertaining to the color development as the indicating material in the sample for quantification is contained and the other compound is preserved separately.

When the oxidative coloring type chromogen is used as the indicating material, a mole-number of this oxidative coloring type chromogen should be controlled to be less than that of the hydrogen peroxide. When the coupling type chromogen is used as the indicating material, the mole-number of this chromogen should be controlled to be less than that of each of the hydrogen peroxide and said the other compound.

The hydrogen peroxide to be used for converting the oxidative coloring type chromogen into the pigment may be hydrogen peroxide itself and it may be generated directly or indirectly from other materials by using enzyme as well. A combination of the material and the enzyme for generating hydrogen peroxide directly or indirectly may include such ones as: cholesterol and cholesterol oxidase; uric acid and uricase; triglyceride and lipoprotein lipase & glycerol oxidase; free fatty acid and acyl-CoA synthetase & acyl-CoA oxidase; glucose and pyranose oxidase; phospholipid and phospholipase D & choline oxidase; creatine and creatinase & sarcosine oxidase; creatinine and creatininase, creatinase & sarcosine oxidase; lactic acid and lactose oxidase; inorganic phosphorous and purine nucleotide phosphorylase & xanthine oxidase; 2,4-dimethoxy-benzoyl-choline and cholinesterase & choline oxidase; allyllamine and monoamine oxidase; and so on.

A reagent chemical for converting the oxidative coloring type chromogen as the indicating material in the sample for quantification into the pigment may be preserved as a single-reagent system or as a plural-reagent system. A preservation as the plural-reagent system is preferable and that as two-reagent system is more preferable. In the case where hydrogen peroxide itself is used, the two-reagent system is preferable which avoids a coexistence of hydrogen peroxide and the active material of peroxide such as peroxidase. In the case where hydrogen peroxide is generated directly or indirectly from other materials by using enzyme, the two-reagent system is preferable which avoids a coexistence of the enzyme to be reacted with the material directly and the material itself. Preferred embodiments of preserving manners of the reagent chemical for converting the oxidative coloring type chromogen into the pigment will be described below. However, it is to be apprehended that the preserving manner is not limited to those embodiments below.

The chromogen in the sample for quantification: N-(2-hydroxy-3-sulfopropyl)-3,5-dimethoxyaniline sodium salt (HSDA)

A first reagent: A reagent prepared by subtracting HSDA from a first reagent of Determiner GL-E (reagent for measuring glucose: provided by Kyowa-medex Corp.) plus glucose.

A second reagent: A second reagent of Determiner GL-E

There is no special limitation on the element to be quantified, but the elements in the blood serum may be preferably included therein. Further, although the quantifying operation of the elements to be quantified is not limited to special manner but can be performed by a general method established as a quantifying method for the elements to be quantified, such a quantifying method is preferable that is not affected substantially by the indicating material.

The elements to be quantified and an example of the measuring method therefor will be described below with the latter indicated in parenthesis. Total protein (biuret method), GOT (JSCC method), GPT (JSCC method), L-lactate dehydro-genase (SSCC method), $\gamma$-GTP (JSCC method), creatinine kinase (IFCC method), cholinesterase (p-HBC method), HDL cholesterol (enzyme method), LDL cholesterol (enzyme method), triglyceride (enzyme method), urea nitrogen (enzyme method), creatinine (enzyme method), uric acid (enzyme method), glucose (enzyme method), alkaline phosphatase (GSCC method), ammonium (enzyme method), sialic acid (enzyme method), ceruloplasmin (colorimetric method), free cholesterol (enzyme method), free fatty acid (enzyme method), lactic acid (enzyme method), lipase (enzyme method), inorganic phosphorus (enzyme method), and monoamine oxidase (enzyme method).

A container equipped with a closable opening/closing means for adding the biological sample thereinto with a specified volume of above-described aqueous solution precisely contained therein is used as the container for collecting and preserving the biological sample or for preparing the sample for quantification. Thus, the test subject can add a proper volume of biological sample such as his/her blood collected by himself /herself without any help of the specifically qualified person such as the doctor, the nurse and the clinical inspection engineer or the expert engineer, without any treatment, for example, as the whole blood and without quantifying the volume thereof one by one, to the container with the specified volume of aqueous solution filled therein, and after having applied thereto a means for preventing a possible vaporization and leakage of the solution contained therein, send to the proper organization (inspection center or hospital) to ask said organization the quantification of the elements which he/she wants to be quantified.

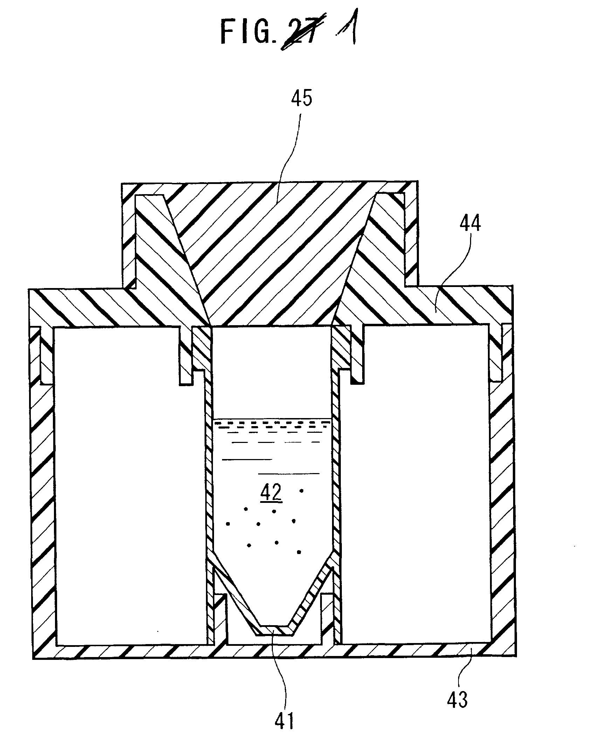

There is no special limitation on the container so far as it has a closable opening/closing means capable of preventing vaporization and leakage of the aqueous solution, and, for example, a reagent bottle with screw cap may be suitable therefor. A preferred embodiment of such container is shown in Fig. 1, which shows a container for preserving the biological sample or for preparing the sample for quantification according to the present invention, which is equipped with a closable opening/closing means for adding the biological sample thereinto. The container will now be described below.

This container comprises a container 41 for collecting a biological sample into which the biological sample is to be added actually, a housing container 43 for receiving the container for collecting the biological sample, said housing container 43 being detachable and being used for stably holding said container 41 for collecting the biological sample, an inner cap 44 for stably fixing said housing container 43 for receiving the container for collecting the biological sample, and an outer cap 45 for stably fixing said container 41 for collecting the biological sample and said inner cap 44. The container 41 for collecting the biological sample contains a specified volume of aqueous solution 42. Further, a specified amount of indicating material is contained in said aqueous solution 42. Thus, the container 41 for collecting the biological sample can be used also as a container for preparing the sample for quantification. The container 41 for collecting the biological sample has no special limitation on a shape thereof, but preferably it may have a shape allowing it to be mounted directly on a turn table of an automatic analyzer. The housing container 43 for receiving the container for collecting the biological sample fixedly holds the container 41 for collecting the biological sample to prevent a falling of the container 41 and a leakage of the aqueous solution 42 possibly caused thereby. Accordingly, the housing container 43 is preferably configured into a shape having both a portion for receiving the container 41 and a portion for securing a positional stability of the whole container. The inner cap 44 and the outer cap 45 serve to close the container 41 and the housing container 43 and thereby confine the aqueous solution 42 in the container 41 for collecting the biological sample thereinto. The inner cap 44 is preferably configured into a shape having a function to fit tightly with each of the housing container 43 and the outer cap 45. The outer cap 45 is preferably configured into a shape having an opening/closing function and also having a portion to fit with the inner cap 44 tightly and a portion to close the container 41 thereby preventing the vaporization and the leakage of the solution 42. It is to be noted that the volume of the aqueous solution 42 to be contained in the container 41 is not limited to the special value, but preferably it is 100 to 5000 $\mu$L and more preferably 200 to 2000 $\mu$L.

Some examples of experiment according to the present invention will now be shown below, but it is to be apprehended that the present invention does not limited to those examples. The reagents and the enzymes used for the experiments are shown below:

Reagent for quantifying glucose: Determiner GL-E (Kyowa-medex Corp.), Glucose reference solution (200mg/dL, A&T Corp.), Isotonic sodium chloride solution (0.9%, prepared in the laboratory), and Ion exchange water (2 $\mu$S/cm or lower, Organo Corp.)

EMBODIMENTS

Embodiment No. 1: Dilution ratio calculation of the blood serum in the case where HSDA is used as the indicating material

Eleven pieces of test tubes (diameter: 10mm; length: 73mm) were prepared, and a 1000 $\mu$L of aqueous solution containing HSDA prepared by double-diluting the reagent 1-B of Determiner GL-E with the ion exchange water was precisely divided and poured into respective test tubes. Then a human pooled serum (prepared from ten person's serum through 3000rpm centrifugal separation processing to be pooled and preserved) was, as shown in Table 1, precisely divided by a unit of 10 $\mu$L and poured into the test tubes Nos. 1 to 11. After having been poured into respective test tubes, they were stirred for five minutes by a mixer (AUTOMATIC LAB MIXER MODEL TH-2).

Table 1

| Test tube No. | Added human serum ($\mu$L) | Theoretical dilution ratio |
|---|---|---|
| 1 | 10 | 101.0 |
| 2 | 20 | 51.0 |
| 3 | 40 | 26.0 |
| 4 | 60 | 17.7 |
| 5 | 80 | 13.5 |
| 6 | 100 | 11.0 |
| 7 | 120 | 9.3 |
| 8 | 140 | 8.1 |
| 9 | 160 | 7.3 |
| 10 | 180 | 6.6 |
| 11 | 200 | 6.0 |

HSDA in the aqueous solution containing HSDA and HSD in the eleven samples described in Table 1 were guided to a pigment by using Determiner GL-E, and the absorptivity thereof was measured. In specific, the aqueous solution containing HSDA and each of the eleven samples (5 $\mu$L) described in Table 1 were mixed with 40 mg/dL glucose aqueous solution (50 $\mu$L) and warmed at 37 °C for five minutes, and then, to this mixture was added the reagent (50 $\mu$L) prepared by adding the whole amount of reagent 2-B (enzyme agent dissolving liquid) of Determiner GL-E to the reagent 2-A (enzyme agent) of the same, and further, the mixture was warmed at 37 °C for five minutes, and after that the absorptivity of the generated pigment was measured at a dominant wavelength of 596 nm and at a sub-wavelength of 884 nm by an automatic analyzer Bio Majesty JCA-BA1650 (provided by JEOL Corp.). The absorptivity $E_1$ in the case of the aqueous solution containing HSDA being used was 0.4486 (= 4486 x $10^{-4}$), and the absorptivity of the samples Nos. 1 to 11 was such as shown in Table 2.

The dilution ratio [= $E_1 / (E_1 - E_2)$] was calculated based on the $E_1$ value and the respective $E_2$ values and compared with the theoretical dilution ratio. The result thereof is shown in Table 2.

Table 2

| Test tube No. | Theoretical dilution ratio | $E_2$ (x $10^4$) | $E_1 - E_2$ (x $10^4$) | Calculated dilution ratio | Coincidence rate (%) |
|---|---|---|---|---|---|
| 1 | 101.0 | 4455 | 31 | 144.7 | 143.3 |
| 2 | 51.0 | 4397 | 89 | 50.4 | 98.8 |
| 3 | 26.0 | 4314 | 172 | 26.1 | 100.3 |
| 4 | 17.7 | 4243 | 243 | 18.5 | 104.5 |
| 5 | 13.5 | 4162 | 324 | 13.8 | 102.6 |
| 6 | 11.0 | 4101 | 385 | 11.7 | 105.9 |
| 7 | 9.3 | 4012 | 474 | 9.5 | 101.4 |
| 8 | 8.1 | 3949 | 537 | 8.4 | 102.6 |
| 9 | 7.3 | 3858 | 628 | 7.1 | 98.5 |
| 10 | 6.6 | 3803 | 683 | 6.6 | 100.2 |
| 11 | 6.0 | 3747 | 739 | 6.1 | 101.2 |

Thus, with an exception of the test tube No. 1, the calculated dilution ratio well coincides with the theoretical dilution ratio within the range of the theoretical dilution ratio of 6.0 to 51.0.

Embodiment No. 2: Quantification of Total protein (TP) in the diluted blood serum by the biuret method

The dilution ratio of the blood serum using the acid blue 9 (brilliant blue FCF) as the indicating material was calculated, and the total protein (TP) in said diluted blood serum was quantified by the biuret method to quantify the total protein (TP) in said blood serum.

HEPES buffer solution (pH 7.7) of 0.1 mol/L concentration containing the acid blue 9 (brilliant blue FCF) was prepared as the aqueous solution containing the indicating material. This solution was divided and poured into ten test tubes by precisely 1000 $\mu$L for each test tube. Then, the human's blood serum was added to respective test tubes by at first 50 $\mu$L then increasing by 50 $\mu$L for each test tube as shown in Table 3 to prepare Nos. 1 to 10 samples for quantification.

The absorptivity ($E_1$) of the HEPES buffer solution containing the acid blue 9 before adding the blood serum thereto and the absorptivity ($E_2$) of the sample for quantification with the blood serum having added thereto were measured at 630 nm. The dilution ratio was calculated based on the $E_1$ value and the $E_2$ value. Further, the total protein (TP) in the sample for quantification of the test tubes Nos. 1 to 10 was quantified by the biuret method. Further, the human's serum value was calculated based on the dilution ratio and the total protein (TP) value for each test sample, and the coincidence rate with the total protein (TP) value calculated by using the blood serum was determined. It is to be noted that the $E_1$ value (the value multiplied by $10^4$) was 870 and the total protein (TP) value quantified by using the blood serum directly was 6.9 g/dL. The result is shown in Table 3.

Table 3

| Test tube No. | Added serum ($\mu$L) | $E_2$ (x $10^4$) | Theoretical dilution ratio | Calculated dilution ratio | Measured concentration (g/dL) | Calculated concentration (g/dL) | Coincidence rate (%) |
|---|---|---|---|---|---|---|---|
| 1 | 50 | 817 | 21.0 | 16.4 | 0.4 | 6.6 | 95.2 |
| 2 | 100 | 784 | 11.0 | 10.1 | 0.7 | 7.1 | 102.6 |
| 3 | 150 | 756 | 7.7 | 7.6 | 0.9 | 6.9 | 99.5 |
| 4 | 200 | 724 | 6.0 | 6.0 | 1.2 | 7.2 | 103.6 |
| 5 | 250 | 699 | 5.0 | 5.1 | 1.5 | 7.6 | 110.6 |
| 6 | 300 | 671 | 4.3 | 4.4 | 1.6 | 7.0 | 101.4 |
| 7 | 350 | 647 | 3.9 | 3.9 | 1.8 | 7.0 | 101.8 |

Table continued

| Test tube No. | Added serum ($\mu$L) | $E_2$ (x $10^4$) | Theoretical dilution ratio | Calculated dilution ratio | Measured concentration (g/dL) | Calculated concentration (g/dL) | Coincidence rate (%) |
|---|---|---|---|---|---|---|---|
| 8 | 400 | 624 | 3.5 | 3.5 | 2.0 | 7.1 | 102.5 |
| 9 | 450 | 605 | 3.2 | 3.3 | 2.2 | 7.2 | 104.7 |
| 10 | 500 | 594 | 3.0 | 3.2 | 2.3 | 7.3 | 105.1 |

Thus, because of good coincidence rate for every sample, it has been ascertained that the quantification of the total protein (TP) in the biological sample obtained by using this quantifying method is effective for every dilution ratio within its range of 3 to 21.

Embodiment No. 3: Quantification of the GOT in the diluted blood serum by the JSCC method

The GOT in the ten samples of diluted blood serum with various dilution ratios prepared according to the method described in the Embodiment No. 2 was quantified by the JSCC method. It is to be noted that the $E_1$ value (the value multiplied by $10^4$) was 870 and the GOT quantified by using the blood serum directly was 48U/L. The result is shown in Table 4.

Table 4

| Test tube No. | Added serum ($\mu$L) | $E_2$ (x $10^4$) | Theoretical dilution ratio | Calculated dilution ratio | Measured concentration (U/dL) | Calculated concentration (U/dL) | Coincidence rate (%) |
|---|---|---|---|---|---|---|---|
| 1 | 50 | 817 | 21.0 | 16.4 | 4 | 66 | 136.8 |
| 2 | 100 | 784 | 11.0 | 10.1 | 5 | 51 | 105.4 |
| 3 | 150 | 756 | 7.7 | 7.6 | 6 | 46 | 95.4 |
| 4 | 200 | 724 | 6.0 | 6.0 | 8 | 48 | 99.3 |
| 5 | 250 | 699 | 5.0 | 5.1 | 12 | 61 | 127.2 |
| 6 | 300 | 671 | 4.3 | 4.4 | 13 | 57 | 118.4 |
| 7 | 350 | 647 | 3.9 | 3.9 | 13 | 51 | 105.7 |
| 8 | 400 | 624 | 3.5 | 3.5 | 14 | 50 | 103.2 |
| 9 | 450 | 605 | 3.2 | 3.3 | 14 | 46 | 95.8 |
| 10 | 500 | 594 | 3.0 | 3.2 | 16 | 50 | 105.1 |

Thus, with an exception of the test tube No. 1, good coincidence rate has been observed. Accordingly, it has been ascertained that the quantification of the GOT in the biological sample obtained by using this quantifying method is effective for every dilution ratio within its range of 3 to 11.

Embodiment No. 4: Quantification of the uric acid in the diluted blood serum by the enzyme method

The uric acid in the ten samples of diluted blood serum with various dilution ratios prepared according to the method described in the Embodiment No. 2 was quantified by the enzyme method, that is, after the uric acid had been converted into allantoin and hydrogen peroxide by uricase, the generated hydrogen peroxide was guided to the pigment by peroxidase, 4-aminoantipyrine and F-DAOS to quantify the uric acid in each sample. It is to be noted that the $E_1$ value (the value multiplied by $10^4$) was 870 and the uric acid value quantified by using the blood serum directly was 4.8 mg/dL. The result is shown in Table 5.

Table 5

| Test tube No. | Added serum (μL) | $E_2$ (x $10^4$) | Theoretical dilution ratio | Calculated dilution ratio | Measured concentration (mg/dL) | Calculated concentration (mg/dL) | Coincidence rate (%) |
|---|---|---|---|---|---|---|---|
| 1 | 50 | 817 | 21.0 | 16.4 | 0.2 | 3.3 | 68.4 |
| 2 | 100 | 784 | 11.0 | 10.1 | 0.5 | 5.1 | 105.4 |
| 3 | 150 | 756 | 7.7 | 7.6 | 0.6 | 4.6 | 95.4 |
| 4 | 200 | 724 | 6.0 | 6.0 | 0.8 | 4.8 | 99.3 |
| 5 | 250 | 699 | 5.0 | 5.1 | 0.9 | 4.6 | 95.4 |
| 6 | 300 | 671 | 4.3 | 4.4 | 1.1 | 4.8 | 100.2 |
| 7 | 350 | 647 | 3.9 | 3.9 | 1.2 | 4.7 | 97.5 |
| 8 | 400 | 624 | 3.5 | 3.5 | 1.4 | 5.0 | 103.2 |
| 9 | 450 | 605 | 3.2 | 3.3 | 1.5 | 4.9 | 102.6 |
| 10 | 500 | 594 | 3.0 | 3.2 | 1.6 | 5.0 | 105.1 |

Thus, with an exception of the test tube No. 1, good coincidence rate has been observed. Accordingly, it has been ascertained that the quantification of the uric acid in the biological sample obtained by using this quantifying method is effective for every dilution ratio within its range of 3 to 11.

Embodiment No. 5: Quantification of the total cholesterol in the diluted blood serum by the enzyme method

The total cholesterol in the ten samples of diluted blood serum with various dilution ratios prepared according to the method described in the Embodiment No. 2 was quantified by the enzyme method. That is, after the ester type cholesterol had been hydrolyzed by chemically modified cholesterol esterase, the free cholesterol in the reaction system was converted into cholestenone and hydrogen peroxide by cholesterol oxidase, and then the generated hydrogen peroxide was guided to the pigment by peroxidase, 4-aminoantipyrine and DOSE to quantify the total cholesterol in each sample. It is to be noted that the $E_1$ value (the value multiplied by $10^4$) was 870 and the total cholesterol value quantified by using the blood serum directly was 137 mg/dL. The result is shown in Table 6.

Table 6

| Test tube No. | Added serum (μL) | $E_2$ (x $10^4$) | Theoretical dilution ratio | Calculated dilution ratio | Measured concentration (mg/dL) | Calculated concentration (mg/dL) | Coincidence rate (%) |
|---|---|---|---|---|---|---|---|
| 1 | 50 | 817 | 21.0 | 16.4 | 6 | 98 | 71.9 |
| 2 | 100 | 784 | 11.0 | 10.1 | 12 | 121 | 88.6 |
| 3 | 150 | 756 | 7.7 | 7.6 | 18 | 137 | 100.3 |
| 4 | 200 | 724 | 6.0 | 6.0 | 23 | 137 | 100.0 |
| 5 | 250 | 699 | 5.0 | 5.1 | 28 | 142 | 104.0 |
| 6 | 300 | 671 | 4.3 | 4.4 | 32 | 140 | 102.1 |
| 7 | 350 | 647 | 3.9 | 3.9 | 36 | 140 | 102.5 |
| 8 | 400 | 624 | 3.5 | 3.5 | 40 | 141 | 103.3 |
| 9 | 450 | 605 | 3.2 | 3.3 | 43 | 141 | 103.0 |
| 10 | 500 | 594 | 3.0 | 3.2 | 44 | 139 | 101.2 |

Thus, with an exception of the test tube No. 1, good coincidence rate has been observed. Accordingly, it has been ascertained that the quantification of the total cholesterol in the biological sample obtained by using this quantifying method is effective for every dilution ratio within its range of 3 to 11.

Embodiment No. 6

A container which receives 1000 μL of aqueous solution with a composition described below and holds it in a sealed condition and which the test subject uses to collect his/her blood and to prepare the sample for quantification by himself/herself was manufactured as shown in Fig. 1.

| Composition of the aqueous solution | |
| --- | --- |
| HSDA | 1.3 mmol/L |
| HEPES (pH 6.5) | 0.1 mol/L |

Embodiment No. 7

The container which receives 1000 μL of aqueous solution with a composition described below and holds it in a sealed condition and which the test subject uses to collect his/her blood and to prepare the sample for quantification by himself/herself was manufactured as shown in Fig. 1.

| Composition of the aqueous solution | |
| --- | --- |
| Acid blue 9 (brilliant blue FCF) | 0.018 mmol/L |
| HEPES (pH 7.7) | 0.1 mol/L |
| Brij-35 (30%) | 0.29% (v/v) |

**Claims**

1. A method of preparing a sample for quantification from a biological sample, which is to be used for quantifying an element to be quantified in said biological sample;
   said method comprising steps of mixing the biological sample with a specified volume of an aqueous solution, thereby obtaining the sample for quantification;
   **characterised in that** the biological sample is of unknown volume collected without quantifying a volume thereof, and by the steps of:

   measuring an absorptivity of an indicating material in the specified volume of the aqueous solution;
   measuring an absorptivity of the indicating material in the sample for quantification;
   calculating a dilution ratio of the biological sample using the absorptivity of the indicating material in said aqueous solution and the absorptivity of the indicating material in said sample for quantification;
   measuring an absorptivity of said element in the sample for quantification.

2. A method in accordance with claim 1, in which said specified volume of aqueous solution contains a specified amount of indicating material.

3. A method in accordance with claim 1, further comprising a step of adding a specified volume of aqueous solution containing a specified amount of indicating material.

4. A method in accordance with claim 2 or claim 3, in which said indicating material is a pigment or a chromogen.

5. A method in accordance with claim 4, in which said chromogen is an oxidative coloring type chromogen.

6. A method in accordance with any of claims 1 to 5, in which said biological sample is either one of a whole blood, a blood plasma or a blood serum.

7. A method in accordance with any of claims 1 to 6, in which said aqueous solution is a buffer solution.

8. A method in accordance with any of claims 1 to 7, in which said elements to be quantified are elements in the blood serum.

9. A method in accordance with claim 1, further comprising the step of obtaining a quantified value of the element in the biological sample using the measured absorptivity of said element and the calculated dilution ratio.

**10.** A quantifying method for quantifying elements to be quantified in a biological sample, said method **characterized by** using a sample for quantification prepared by a preparing method according to claim 1.

**11.** A quantifying method in accordance with claim 10, in which said unknown volume of biological sample collected without quantifying a volume thereof is mixed with a specified volume of aqueous solution.

**12.** A quantifying method in accordance with either of claim 10 or 11, said method **characterized in** further comprising the steps of:

determining a dilution ratio of said biological sample in said sample for quantification; and
quantifying a concentration of elements to be quantified in said sample for quantification.

**13.** A quantifying method, said method comprising the steps of:

1) preparing a sample for quantification according to claim 1;
2) wherein the step of calculating a dilution ratio comprises determining a dilution ratio (a) of said biological sample from a concentration ($C_1$) of the indicating material in said specified volume of aqueous solution containing said specified amount of indicating material and a concentration ($C_2$) of the indicating material in said sample for quantification;
3) determining a concentration (Y) of the elements to be quantified in said sample for quantification; and
4) determining the elements to be quantified in the biological sample based on the dilution ratio (a) of the biological sample determined in the step 2) and said concentration (Y) of the elements to be quantified in the sample for quantification determined in the step 3).

**14.** A quantifying method in accordance with claim 13, in which said aqueous solution is a buffer solution.

**15.** A method in accordance with claim 13, in which said indicating material is a pigment or a chromogen.

**16.** A method in accordance with claim 14, in which said chromogen is an oxidative coloring type chromogen.

**17.** A method in accordance with either of claim 15 or 16, in which an absorptivity ($E_1$) of said specified volume of aqueous solution containing said specified amount of indicating material and an absorptivity ($E_2$) of said sample for quantification are used in substitution for $C_1$ and $C_2$ respectively.


**Patentansprüche**

**1.** Verfahren zum Herstellen einer Probe für die quantitative Bestimmung aus einer biologischen Probe, die für das quantitative Bestimmen eines Elements verwendet werden soll, das in der biologischen Probe quantitativ bestimmt werden soll;
wobei das genannte Verfahren die Schritte umfasst:

Mischen der biologischen Probe mit einem vorgegebenen Volumen einer wäßrigen Lösung, wodurch die Probe für die quantitative Bestimmung erhalten wird;

**dadurch gekennzeichnet, dass** die biologische Probe ein unbekanntes Volumen aufweist, und ohne quantitative Bestimmung von deren Volumen gesammelt wird, und durch die Schritte:

Messen eines Absorptionsvermögens eines indizierenden Materials in dem vorgegebenen Volumen der wäßrigen Lösung;
Messen eines Absorptionsvermögens des indizierenden Materials in der Probe für die quantitative Bestimmung;
Berechnen eines Verdünnungsverhältnisses der biologischen Probe, indem das Absorptionsvermögen des indizierenden Materials in der genannten wäßrigen Lösung, und das Absorptionsvermögen des indizierenden Materials in der genannten Probe für die quantitative Bestimmung verwendet wird;
Messen eines Absorptionsvermögen des Elements in der Probe für die quantitative Bestimmung.

**2.** Verfahren gemäß Anspruch 1, bei dem das vorgegebene Volumen einer wäßrigen Lösung eine vorgegebene Menge des indizierenden Materials enthält.

**3.** Verfahren gemäß Anspruch 1, desweiteren umfassend einen Schritt des Dazugebens eines vorgegebenen Volumens einer wäßrigen Lösung, die eine vorgegebene Menge des indizierenden Materials enthält.

**4.** Verfahren gemäß Anspruch 2 oder Anspruch 3, bei dem das indizierende Material ein Pigment oder ein Chromogen ist.

**5.** Verfahren gemäß Anspruch 4, bei dem das Chromogen ein vom oxidativ farberzeugenden Typ ist.

**6.** Verfahren gemäß einem der Ansprüche 1 bis 5, bei dem die biologische Probe entweder eine Probe von Vollblut, von Blutplasma oder von Blutserum ist.

**7.** Verfahren gemäß einem der Ansprüche 1 bis 6, bei dem die wäßrige Lösung eine Pufferlösung ist.

**8.** Verfahren gemäß einem der Ansprüche 1 bis 7, bei dem die Elemente, die quantitativ bestimmt werden sollen, Elemente in dem Blutserum sind.

**9.** Verfahren gemäß Anspruch 1, desweiteren umfassend den Schritt des Erhaltens eines quantitativ bestimmten Werts des Elements in der biologischen Probe, indem das gemessene Absorptionsvermögen des Elements und des berechneten Verdünnungsverhältnisses verwendet wird.

**10.** Quantifizierendes Verfahren für quantitativ zu bestimmende Elemente, die in einer biologischen Probe quantitativ bestimmt werden sollen, wobei das Verfahren durch das Verwenden einer Probe für die quantitative Bestimmung, die durch ein Herstellungsverfahren gemäß Anspruch 1 hergestellt wird, **gekennzeichnet** ist.

**11.** Quantifizierendes Verfahren gemäß Anspruch 10, bei dem das unbekannte Volumen einer biologischen Probe, die ohne quantitatives Bestimmen von deren Volumen gesammelt wird, mit einem vorgegebenem Volumen einer wäßrigen Lösung gemischt wird.

**12.** Quantifizierendes Verfahren gemäß einem der Ansprüche 10 oder 11, wobei das Verfahren **dadurch gekennzeichnet ist, dass** es die weiteren Schritte umfasst:

Bestimmen eines Verdünnungsverhältnisses der biologischen Probe in der Probe für die quantitative Bestimmung; und
quantitatives Bestimmen einer Konzentration von Elementen, die in der Probe für die quantitative Bestimmung quantitativ bestimmt werden soll.

**13.** Quantifizierendes Verfahren, wobei das Verfahren die Schritte umfasst:

1) Herstellen einer Probe für die quantitative Bestimmung gemäß Anspruch 1;
2) wobei der Schritt des Berechnens eines Verdünnungsverhältnisses das Bestimmen eines Verdünnungsverhältnisses (a) der biologischen Probe mit einer Konzentration ($C_1$) des indizierenden Materials in dem vorgegebenen Volumen einer wäßrigen Lösung, welche die vorgegebene Menge an indizierendem Material enthält und einer Konzentration ($C_2$) des indizierenden Materials in der Probe für die quantitative Bestimmung umfasst;
3) Bestimmen einer Konzentration (Y) der Elemente, die in der Probe für die quantitative Bestimmung quantitativ bestimmt werden sollen; und
4) Bestimmen der Elemente, die in der biologischen Probe quantitativ bestimmt werden sollen, basierend auf dem Verdünnungsverhältnis (a) der biologischen Probe, bestimmt in dem Schritt 2), und der Konzentration (Y) der Elemente, die quantitativ in der Probe für die quantitative Bestimmung, bestimmt in dem Schritt 3, bestimmt werden soll.

**14.** Quantifizierendes Verfahren gemäß Anspruch 13, bei dem die wäßrige Lösung eine Pufferlösung ist.

**15.** Verfahren gemäß Anspruch 13, bei dem das indizierende Material ein Pigment oder ein Chromogen ist.

**16.** Verfahren gemäß Anspruch 14, bei dem das Chromogen ein Chromogen vom oxidativ farberzeugenden Typ ist.

**17.** Verfahren gemäß einem der Ansprüche 15 oder 16, bei dem ein Absorptionsvermögen ($E_1$) des vorgegebenen Volumens der wäßrigen Lösung, welche die vorgegebene Menge an indizierendem Material enthält, und ein Absorptionsvermögen ($E_2$) der Probe für die quantitative Bestimmung als Ersatz für $C_1$ beziehungsweise $C_2$ verwendet werden.

**Revendications**

**1.** Procédé de préparation d'un échantillon pour quantification à partir d'un échantillon biologique, qui est destiné à être utilisé pour quantifier un élément à quantifier dans ledit échantillon biologique ;
ledit procédé comprenant les étapes consistant à mélanger l'échantillon biologique avec un volume spécifique d'une solution aqueuse, en obtenant ainsi l'échantillon pour quantification ;
**caractérisé en ce que** l'échantillon biologique est d'un volume inconnu recueilli sans quantification de son volume, et par les étapes suivantes :

mesure d'une absorptivité d'une matière indicatrice dans le volume spécifié de la solution aqueuse ;
mesure d'une absorptivité de la matière indicatrice dans l'échantillon pour quantification ;
calcul d'un rapport de dilution de l'échantillon biologique en utilisant l'absorptivité de la matière indicatrice dans ladite solution aqueuse et l'absorptivité de la matière indicatrice dans ledit échantillon pour quantification ;
mesure d'une absorptivité dudit élément dans l'échantillon pour quantification.

**2.** Procédé suivant la revendication 1, dans lequel ledit volume spécifié de solution aqueuse contient une quantité spécifiée de matière indicatrice.

**3.** Procédé suivant la revendication 1, comprenant en outre une étape d'addition d'un volume spécifié de solution aqueuse contenant une quantité spécifiée de matière indicatrice.

**4.** Procédé suivant la revendication 2 ou la revendication 3, dans lequel ladite matière indicatrice est un pigment ou un agent chromogène.

**5.** Procédé suivant la revendication 4, dans lequel ledit agent chromogène est un agent chromogène de type colorant à oxydatif.

**6.** Procédé suivant l'une quelconque des revendications 1 à 5, dans lequel ledit échantillon biologique est un des échantillons consistant en un échantillon de sang entier, un échantillon de plasma sanguin et un échantillon de sérum sanguin.

**7.** Procédé suivant l'une quelconque des revendications 1 à 6, dans lequel ladite solution aqueuse est une solution tampon.

**8.** Procédé suivant l'une quelconque des revendications 1 à 7, dans lequel lesdits éléments à quantifier sont des éléments dans le sérum sanguin.

**9.** Procédé suivant la revendication 1, comprenant en outre l'étape d'obtention d'une valeur quantifiée de l'élément dans l'échantillon biologique en utilisant l'absorptivité mesurée dudit élément et le rapport de dilution calculé.

**10.** Méthode de quantification pour quantifier des éléments à quantifier dans un échantillon biologique, ladite méthode étant **caractérisée par** l'utilisation d'un échantillon pour quantification préparée par un procédé de préparation suivant la revendication 1.

**11.** Méthode de quantification suivant la revendication 10, dans laquelle ledit volume inconnu d'échantillon biologique recueilli sans quantification de son volume est mélangé à un volume spécifié de solution aqueuse.

**12.** Méthode de quantification suivant l'une des revendications 10 et 11, ladite méthode étant **caractérisée en ce qu'**elle comprend en outre les étapes suivantes :

détermination d'un rapport de dilution dudit échantillon biologique dans ledit échantillon pour quantification ; et
quantification d'une concentration d'éléments à quantifier dans ledit échantillon pour quantification.

**13.** Procédé de quantification, ladite méthode comprenant les étapes suivantes :

1) préparation d'un échantillon pour quantification suivant la revendication 1 ;
2) l'étape de calcul d'un rapport de dilution comprend la détermination d'un rapport de dilution (a) dudit échantillon biologique à partir d'une concentration ($C_1$) de la matière indicatrice dans ledit volume spécifié de solution aqueuse contenant ladite quantité spécifiée de matière indicatrice, et d'une concentration ($C_2$) de la matière indicatrice dans ledit échantillon pour quantification ;
3) détermination d'une concentration (Y) des éléments à quantifier dans ledit échantillon pour quantification ; et
4) détermination des éléments à quantifier dans l'échantillon biologique sur la base du rapport de dilution (a) de l'échantillon biologique déterminé dans l'étape 2) et de ladite concentration (Y) des éléments à quantifier dans l'échantillon pour quantification déterminée dans l'étape 3).

**14.** Méthode de quantification suivant la revendication 13, dans laquelle ladite solution aqueuse est une solution tampon.

**15.** Méthode suivant la revendication 13, dans laquelle ladite matière indicatrice est un pigment ou un agent chromogène.

**16.** Méthode suivant la revendication 14, dans laquelle ledit agent chromogène est un agent chromogène de type colorant à oxydatif.

**17.** Méthode suivant l'une des revendications 15 et 16, dans lequel une absorptivité ($E_1$) dudit volume spécifié de solution aqueuse contenant ladite quantité spécifiée de matière indicatrice, et une absorptivité ($E_2$) dudit échantillon pour quantification sont utilisées respectivement en remplacement de $C_1$ et $C_2$.

# FIG. 27 1